# EUROPÄISCHE PATENTSCHRIFT

(11) **EP 1 403 248 B1**
(45) Veröffentlichungstag und Bekanntmachung des Hinweises auf die Patenterteilung: **24.05.2006**
(21) Anmeldenummer: 03020535.5
(22) Anmeldetag: 17.09.2003
(51) Int. Cl.: C07C 263/10, C07C 265/14

(54) **Verfahren zum Quenchen eines gasförmigen Reaktionsgemisches bei der Gasphasenphosgenierung von Diaminen zu Diisocyanaten**
Process for the preparation of diisocyanates by quenching the gas phase resulting from the phosgenation of the corresponding diamines
Procédé de préparation de composes du type diisocyanates par trempe de la phase gazeuse résultant de la phosgénation de diamines

(30) Priorität: 30.09.2002 DE 10245704
(43) Veröffentlichungstag der Anmeldung: 31.03.2004
(73) Patentinhaber: Bayer MaterialScience AG, 51368 Leverkusen (DE)
(72) Erfinder: Herold, Heiko, 41470 Neuss (DE); Stutz, Herbert, Dr., 41541 Dormagen (DE); Brockhaus, Hans-Joachim, 51375 Leverkusen (DE); Michele, Volker, 51065 Köln (DE)

(56) Entgegenhaltungen:
- US-A- 5 633 396

## Beschreibung

Die Erfindung betrifft ein Verfahren zum Quenchen eines gasförmigen Reaktionsgemisches bei der Phosgenierung von Diaminen in der Gasphase zur Herstellung von Diisocyanaten, wobei das Gasgemisch wenigstens Diisocyanat, Phosgen und Chlorwasserstoff enthält. Das Quenchen erfolgt durch Eindüsen einer Quench-Flüssigkeit in das Gasgemisch.

Die Herstellung von Diisocyanaten durch Umsetzung von Diaminen mit Phosgen in der Gasphase wird beispielsweise in der EP-A 0 289 840 beschrieben. Die in einem zylinderförmigen Reaktionsraum, beispielsweise einem Rohrreaktor, gebildeten Diisocyanate sind bei den Reaktionstemperaturen von 300 bis 500°C thermisch nicht stabil. Eine rasche Abkühlung der Reaktionsgase nach der Phosgenierungsreaktion auf Temperaturen unter 150°C ist daher notwendig, um die Bildung unerwünschter Nebenprodukte durch den thermischen Zerfall von Diisocyanat oder durch eine Weiterreaktion zu vermeiden. Dazu wird in der EP-A 0 289 840 das den Reaktionsraum kontinuierlich verlassende gasförmige Gemisch, welches u.a. Diisocyanat, Phosgen und Chlorwasserstoff enthält, in ein inertes Lösungsmittel, z.B. Dichlorbenzol, eingeleitet. Nachteilig bei diesem Verfahren ist, dass die Strömungsgeschwindigkeit, mit der das Gasgemisch durch das Lösungsmittelbad geleitet wird, relativ niedrig gewählt werden muss, da bei zu hohen Geschwindigkeiten Lösungsmittel und die darin gelösten Verbindungen mitgerissen würden. In einem nachfolgenden Schritt müssten die flüssigen Verbindungen vom Gas abgetrennt werden. Ein weiterer Nachteil ist, dass aufgrund der niedrigen Strömungsgeschwindigkeit und eines geringen Wärmeübergangsterms große Lösungsmittelbehälter eingesetzt werden müssen, um die Abkühlung zu erzielen.

Weiterhin bekannt sind Verfahren, die zur Abkühlung der Reaktionsgase Wärmetauscher einsetzen und/oder die Gase in Vakuum entspannen. Der Nachteil von Wärmetauschern liegt darin, dass wegen des schlechten Wärmeübergangs große Austauschflächen, und damit große Wärmetauscher, für eine effektive Kühlung benötigt werden. Außerdem kommt es zu Ablagerungen von Feststoffen auf den verhältnismäßig kalten Flächen der Wärmetauscher durch Nebenreaktionen des Gasgemisches auf diesen Flächen, wie z.B. Zersetzung oder Polymerisation. Der Wärmeübergang wird dadurch weiter verschlechtert, was eine höhere Verweilzeit und damit wiederum eine weitere Zunahme der Nebenproduktbildung zur Folge hat. Darüber hinaus ergeben sich durch die Reinigung der Abkühlungsstufe unerwünschte Stillstandszeiten für die Gesamtanlage.

Der Erfindung liegt die Aufgabe zugrunde, unter Vermeidung der genannten Nachteile das gasförmige Reaktionsgemisch bei der Gasphasenphosgenierung von Diaminen zur Herstellung von Diisocyanaten schnell auf eine Temperatur abzukühlen, bei der das jeweilige Reaktionsprodukt thermisch stabil ist. Dabei soll gleichzeitig die Bildung von unerwünschten Nebenprodukten unterbunden werden.

Die Lösung der Aufgabe erfolgt erfindungsgemäß durch die Merkmale des Anspruchs 1.

Gegenstand der Erfindung ist ein Verfahren zum Quenchen eines gasförmigen Reaktionsgemisches bei der Phosgenierung von Diaminen in der Gasphase zur Herstellung von Diisocyanaten, wobei das gasförmige Reaktionsgemisch wenigstens ein Diisocyanat, Phosgen und Chlorwasserstoff umfasst, durch Eindüsen einer Quench-Flüssigkeit in das kontinuierlich aus einer zylinderförmigen Reaktionszone in die nachgeschaltete zylinderförmige Quench-Zone strömende Gasgemisch, wobei die Quench-Flüssigkeit mit Hilfe von mindestens zwei Sprühdüsen, welche am Eingang der Quench-Zone in gleichen Abständen entlang des Umfangs der Quench-Zone angeordnet sind und einen Öffnungsminkel von 30 bis 60° and aufweisen, eingedüst wird.

Das gasförmige Reaktionsgemisch kann neben Phosgen, Chlorwasserstoff und dem hauptsächlich gebildeten Diisocyanat weitere Isocyanate, welche als Nebenprodukte entstehen, sowie Stickstoff und/oder organische Lösungsmittel umfassen.

Beispiele für Diisocyanate, die durch Gasphasenphosgenierung von Diaminen hergestellt werden, sind: Hexamethylendiisocyanat (HDI), Isophorondiisocyanat (IPDI), Naphthylendiisocyanat (NDI), Toluylendiisocyanat (TDI), Diphenylmethandiisocyanat, Dicyclohexylmethandiisocyanat (HMDI).

Der Vorteil des erfindungsgemäßen Verfahrens liegt darin, dass durch das Versprühen einer geeigneten Quench-Flüssigkeit die gewünschte rasche Abkühlung des Gasgemisches, welches ein Diisocyanat, Chlorwasserstoff und überschüssiges Phosgen umfasst, beim Verlassen des Reaktors von 300 bis 400°C auf maximal 150°C erzielt wird. Die Kontaktzeit, in der die Abkühlung erfolgt, beträgt dabei von 0,2 bis 3 s.

Das Versprühen der Flüssigkeit erfolgt mit üblichen Sprühdüsen oder durch Öffnungen, beispielsweise Schlitze oder Löcher, am Ausgang der Reaktionszone bzw. Eingang der Quench-Zone. Sind nur zwei Sprühdüsen vorgesehen, so sind diese diametral angeordnet. Bei den Sprühdüsen kann es sich um Einzeldüsen handeln. Vorzugsweise werden jedoch Düsenköpfe mit jeweils mindestens zwei Einzeldüsen eingesetzt, wobei bevorzugt Einstoffdüsen gewählt werden.

Ein weiterer Vorteil des Verfahrens ist, dass die Quench-Flüssigkeit so in den Gasstrom eingesprüht wird, dass das heiße Reaktionsgas die relativ kalten Flächen der Quench-Zone oder der Düsen und deren Zuleitungen nicht kontaktiert. Erst wenn die Gasmischung auf den für das jeweilige Diisocyanat stabilen Temperaturbereich abgekühlt ist, kommt sie in Berührung mit den relativ kalten Wänden der Quench-Zone oder anderen Bauteilen.

Die Sprühdüsen sind bevorzugt unabhängig voneinander so angeordnet, dass jeweils die Strömungsrichtung der Quench-Flüssigkeit einen Winkel von 0° bis 50°, insbesondere von 20° bis 35°, gegenüber der Strömungsrichtung des Gasgemisches aufweist. Die Strömungsrichtung des Gasgemisches verläuft im Wesentlichen entlang der Achse der zylinderförmigen Reaktionszone bzw. der Quench-Zone. Bei einer senkrechten Anordnung des Rohrreaktors strömt das Gas von oben nach unten durch die Reaktionszone und die nachgeschaltete Quench-Zone. Analog verläuft die Strömungsrichtung der Quench-Flüssigkeit entlang der Achse der Sprühdüse. Der Öffnungswinkel der Sprühdüsen beträgt unabhängig voneinander von 30° bis 60°. In einer besonders bevorzugten Ausführungsform weist die Strömungsrichtung all derjenigen Düsen, die in einer Ebene angeordnet sind, den selben Winkel gegenüber der Strömungsrichtung des Gasgemisches sowie denselben Öffnungswinkel auf.

Als Quench-Flüssigkeit eignen sich organische Lösungsmittel oder eine Mischung verschiedener organischer Lösungsmittel, welche mit dem gebildeten Diisocyanat nicht reagieren. Die Wahl des Lösungsmittels wird u.a. auch von der Löslichkeit des Phosgens bestimmt. Als Lösungsmittel eignen sich beispielsweise Toluol, Chlortoluol, Xylol und Chlomaphthalin. Insbesondere eignen sich Monochlorbenzol und o-Dichlorbenzol. Auch kann eine Lösung des gebildeten Diisocyanats in einem dieser organischen Lösungsmittel verwendet werden. Dabei beträgt der Lösungsmittelanteil vorzugsweise von 40 bis 90 Vol%. Die Temperatur der Quench-Flüssigkeit beträgt vorzugsweise von 100 bis 170°C.

Die der zylinderförmigen Reaktionszone nachgeschaltete Quench-Zone ist ebenfalls zylinderförmig ausgebildet. Dabei kann der Durchmesser der Quench-Zone im Wesentlichen gleich dem der Reaktionszone oder größer als der der Reaktionszone gewählt werden. Bei der Reaktionszone handelt es sich insbesondere um einen Rohrreaktor ohne Einbauten.

Das erfindungsgemäße Verfahren hat den Vorteil, dass die Abkühlung der Reaktionsgase rasch, insbesondere innerhalb von 0,2 bis 3 s, unmittelbar nach Ablauf der Reaktion erfolgt, da der aus dem Reaktor strömende Gasstrom nicht abgebremst und/oder in einen Behälter eingeleitet werden muss, sondern direkt durch einen Strom einer zerstäubten Quench-Flüssigkeit geführt wird. Außerdem ist die Quench-Zone so ausgebildet und sind die Düsen so angebracht, dass das heiße Gasgemisch in der Quench-Zone keine der relativ kalten Flächen kontaktiert. Dazu kann beispielsweise der Durchmesser der zylinderförmigen Quench-Zone größer sein als der Durchmesser der Reaktionszone.

In einer bevorzugten Ausführungsform des Verfahrens kann das Quenchen der Reaktionsgase auch mehrstufig, insbesondere zweistufig, erfolgen. Dabei umfasst jede Quench-Stufe mindestens zwei Sprühdüsen in gleichen Abständen entlang des Umfangs der Quench-Zone. In den verschiedenen Quench-Stufen können gleiche Quench-Flüssigkeiten eingesetzt werden. Besonders bevorzugt werden jedoch beim zweistufigen Quenchen verschiedene Quench-Flüssigkeiten in den beiden Stufen eingesetzt, nämlich ein organisches Lösungsmittel, insbesondere Monochlorbenzol oder o-Dichlorbenzol, in der ersten Stufe und in der zweiten Stufe eine Lösung des gebildeten Diisocyanats in dem organischen Lösungsmittel, welches in der ersten Quench-Stufe verwendet wurde. Vorzugsweise beträgt dabei der Volumenanteil des Lösungsmittels von 40 bis 90 %.

Nachfolgend wird das erfindungsgemäße Verfahren anhand bevorzugter Ausfiihrungsformen unter Bezugnahme auf die anliegenden Figuren näher erläutert. Es zeigen:
- Fig. 1: ein schematischer Querschnitt einer ersten bevorzugten Ausführungsform der Quench-Zone
- Fig. 2: ein schematischer Querschnitt einer zweiten bevorzugten Ausführungsform der Quench-Zone.

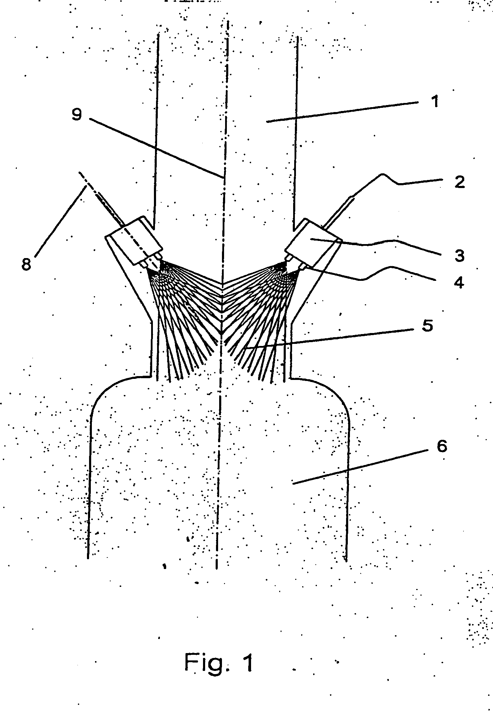

Figur 1 zeigt eine zylinderförmige Reaktionszone 1, die von dem gasförmigen Gemisch von oben nach unten entlang der gestrichelten Linie 9 durchströmt wird. Beim Verlassen der Reaktionszone 1 durchströmt das Gasgemisch eine ebenfalls zylinderförmige Quench-Zone 5. In der Quench-Zone 5 sind zwei Düsenköpfe 3 mit je zwei Einzeldüsen 4 diametral angeordnet. Die Quench-Flüssigkeit wird über eine Zuleitung 2 dem Düsenkopf 3 zugeführt. Die Düsen 4 bzw. der Düsenkopf 3 sind so angeordnet, dass die Strömungsrichtung der Quench-Flüssigkeit (dargestellt durch die gestrichelte Linie 8) und die des Gasstroms 9 einen Winkel von 0° bis 50°, insbesondere von 20° bis 35°, zueinander aufweisen und damit das heiße Gasgemisch die kälteren Düsen bzw. den Düsenkopf nicht kontaktiert. In der Quench-Zone 5 erfolgt die Abkühlung des Reaktionsgases durch Verdampfung der zerstäubten Flüssigkeit. Die verbleibende Flüssigkeit und das abgekühlte Reaktionsgas gelangen in den darunter liegenden Flüssigkeitssammelbehälter 6, der gleichzeitig als Pumpenvorlage und als Trennapparat für Gas und Flüssigkeit dient.

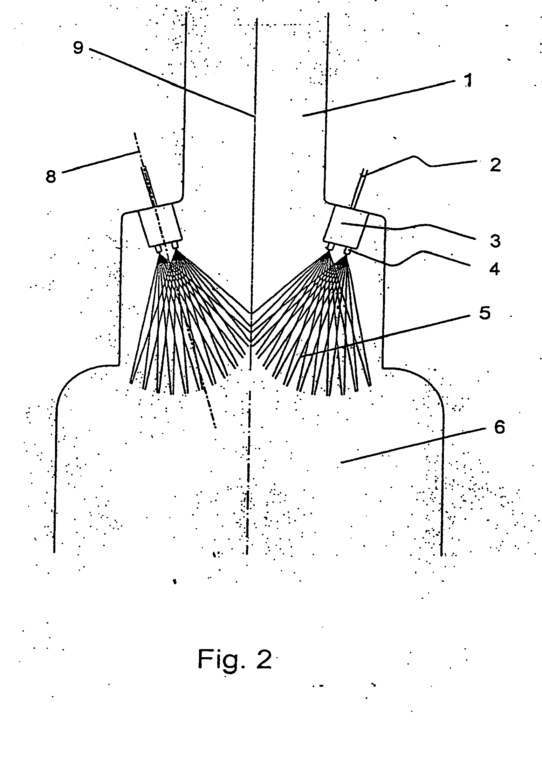

Die in Figur 2 dargestellte Ausführungsform der Quench-Zone entspricht prinzipiell der in Fig. 1 dargestellten Ausführungsform. Gleiche oder ähnliche Bauteile besitzen daher die selben Bezugszeichen wie in Fig.1. Die in Fig. 2 gezeigte Ausführungsform unterscheidet sich von der in Fig. 1 gezeigten dadurch, dass der Durchmesser der Quench-Zone 5 größer als der des Rohrreaktors 1 ist.

### Beispiel

Aus einem senkrecht angeordneten Rohreaktor mit einem Durchmesser von 260 mm strömen 6700 kg/h eines 400°C heißen Gasgemischs aus Isophorondiisocyanat, Chlorwasserstoff und überschüssigem Phosgen bei einem Druck von 1000 bis 1800 mbar mit einer Geschwindigkeit von 18 m/s in eine nachgeschaltete Quench-Zone mit einem Durchmesser von 510 mm. Die Rohraufweitung vom Reaktor zur Quench-Zone ist mit einem Winkel von 75° gegen die Vertikale ausgeführt. In der Aufweitung sind in gleichen Abständen entlang des Umfangs vier Einzeldüsen eingebracht, die 130·10³ kg/h einer Lösung von Isophorondiisocyanat in Monochlorbenzol in einem Volumenverhältnis von 20:80 und einer Temperatur von 120°C versprühen. Die Strömungsrichtung der Quench-Flüssigkeit beträgt 35° gegenüber der Strömungsrichtung des Gasgemischs. Die Öffnungswinkel der Düsen betragen 30°. In der Quench-Zone erfolgt die Abkühlung des Reaktionsgases. Die kondensierbaren Bestandteile werden in der Lösung gelöst und die zur Abkühlung benötigte Menge an Monochlorbenzol verdampft. Das Flüssigkeits-Gas-Gemisch wird in einen Abscheider eingeleitet. Nach einer Kontaktzeit von 0,8 bis 1,3 Sekunden beträgt die Temperatur der im Abscheider gesammelten aufkonzentrierten Isophorondiisocyanatlösung 140°C. Das aus dem Abscheider austretende Gas hat eine Temperatur von 145°C.

## Patentansprüche

1. Verfahren zum Quenchen eines gasförmigen Reaktionsgemisches bei der Phosgenierung von Diaminen in der Gasphase zur Herstellung von Diisocyanaten, wobei das gasförmige Reaktionsgemisch wenigstens ein Diisocyanat, Phosgen und Chlorwasserstoff umfasst, durch Eindüsen einer Quench-Flüssigkeit in das kontinuierlich aus einer zylinderförmigen Reaktionszone (1) in die nachgeschaltete zylinderförmige Quench-Zone (5) strömende Gasgemisch, **dadurch gekennzeichnet, dass** die Quench-Flüssigkeit mit Hilfe von mindestens zwei Sprühdüsen (4), welche am Eingang der Quench-Zone (5) in gleichen Abständen entlang des Umfangs der Quench-Zone (5) angeordnet sind und einen Öffnungsminkel von 30-60° aufweisen, eingedüst wird.

2. Verfahren nach Anspruch 1, **dadurch gekennzeichnet, dass** die Sprühdüsen unabhängig voneinander so angeordnet sind, dass jeweils die Strömungsrichtung der Quench-Flüssigkeit einen Winkel von 0° bis 50°, insbesondere von 20° bis 35°, zu der Strömungsrichtung des Gasgemisches aufweist.

3. Verfahren nach einem der Ansprüche 1 -2, **dadurch gekennzeichnet, dass** die Sprühdüsen aus einem Düsenkopf (3) mit jeweils mindestens zwei Einzeldüsen (4) bestehen.

4. Verfahren nach einem der Ansprüche 1 -3, **dadurch gekennzeichnet, dass** die Quench-Flüssigkeit ein organisches Lösungsmittel, insbesondere Monochlorbenzol oder o-Dichlorbenzol, oder eine Mischung verschiedener organischer Lösungsmittel oder eine Lösung des Diisocyanats in einem organischen Lösungsmittel, insbesondere Monochlorbenzol oder o-Dichlorbenzol, ist.

5. Verfahren nach einem der Ansprüche 1 - 4, **dadurch gekennzeichnet, dass** die Temperatur der Quench-Flüssigkeit von 100 bis 170 °C beträgt.

6. Verfahren nach einem der Ansprüche 1 - 5, **dadurch gekennzeichnet, dass** das Quenchen mehrstufig, insbesondere zweistufig, ausgeführt wird.

7. Verfahren nach Anspruch 6, **dadurch gekennzeichnet, dass** in verschiedenen Quench-Stufen verschiedene Quench-Flüssigkeiten verwendet werden.

## Claims

1. Process for quenching a gaseous reaction mixture during the phosgenation of diamines in the gas phase to produce diisocyanates, in which the gaseous reaction mixture comprises at least a diisocyanate, phosgene and hydrogen chloride, injecting a quenching liquid into the gas mixture continuously flowing out of a cylindrical reaction zone (1) into the downstream cylindrical quenching zone (5), **characterized in that** the quenching liquid is injected by means of at least two spray nozzles (4) arranged at the entrance to the quenching zone (5) at equal distances along the circumference of the quenching zone (5) and having an aperture angle of 30-60°.

2. Process according to claim 1, **characterized in that** the spray nozzles independently of one another are arranged such that the direction of flow of each quenching liquid is at an angle of 0° to 50°, in particular 20° to 35° to the direction of flow of the gas mixture.

3. Process according to either of Claims 1-2, **characterized in that** the spray nozzles comprise a nozzle head (3) with in each case at least two individual nozzles (4).

4. Process according to any of Claims 1-3, **characterized in that** the quenching liquid is an organic solvent, in particular monochlorobenzene or o-dichlorobenzene, or a mixture of different organic solvents or a solution of the diisocyanate in an organic solvent, in particular monochlorobenzene or o-dichlorobenzene.

5. Process according to any of Claims 1-4, **characterized in that** the temperature of the quenching liquid is 100 to 170°C.

6. Process according to any of Claims 1-5, **characterized in that** the quenching is carried out in a plurality of steps, in particular in two steps.

7. Process according to Claim 6, **characterized in that** different quenching liquids are used in differnt quenching steps.

## Revendications

1. Procédé de trempe d'un mélange de réaction gazeux lors de la phosgénation en phase gazeuse de diamines pour la fabrication de diisocyanates, le mélange de réaction gazeux comprenant au moins un diisocyanate, du phosgène et du chlorure d'hydrogène, par pulvérisation d'un liquide de trempe dans le mélange de gaz s'écoulant en continu d'une zone de réaction (1) cylindrique dans la zone de trempe (5) cylindrique d'aval, **caractérisé en ce que** le liquide de trempe est pulvérisé à l'aide d'au moins deux buses de pulvérisation (4), qui sont disposées à l'entrée de la zone de trempe (5) à intervalles réguliers le long de la périphérie de la zone de trempe (5) et présentent un angle d'ouverture de 30 à 60°.

2. Procédé suivant la revendication 1, **caractérisé en ce que** les buses de pulvérisation sont disposées indépendamment l'une de l'autre de telle façon que la direction d'écoulement du liquide de trempe fasse un angle de 0° à 50°, en particulier de 20° à 35°, avec la direction d'écoulement du mélange de gaz.

3. Procédé suivant l'une des revendications 1 - 2, **caractérisé en ce que** les buses de pulvérisation sont constituées d'une tête de pulvérisation (3) comportant respectivement au moins deux buses de pulvérisation individuelles (4).

4. Procédé suivant l'une des revendications 1 - 3, **caractérisé en ce que** le liquide de trempe est un solvant organique, en particulier du monochlorobenzène ou de l'o-dichlorobenzène, ou un mélange de différents solvants organiques ou une solution du diisocyanate dans un solvant organique, en particulier le monochlorobenzène ou l'o-dichlorobenzène.

5. Procédé suivant l'une des revendications 1 - 4, **caractérisé en ce que** la température du liquide de trempe est de 100 à 170 °C.

6. Procédé suivant l'une des revendications 1 - 5, **caractérisé en ce que** la trempe est exécutée en plusieurs étages, en particulier en deux étages.

7. Procédé suivant la revendication 6, **caractérisé en ce qu'**on utilise des liquides de trempe différents dans différents étages de trempe.
